# EUROPÄISCHE PATENTSCHRIFT

(11) **EP 3 268 116 B1**
(45) Veröffentlichungstag und Bekanntmachung des Hinweises auf die Patenterteilung: **30.08.2023**
(21) Anmeldenummer: 16710945.3
(22) Anmeldetag: 11.03.2016
(51) Int. Cl.: B01D 69/10, A61M 1/16, B01D 71/70, B01D 63/06, B01D 63/08, B01D 63/10, B01D 67/00

(54) **MEMBRAN FÜR EINEN OXYGENATOR FÜR GAUSAUSTAUSCH IM BLUTKREISLAUF, OXYGENATOR MIT EINER DERARTIGEN MEMBRAN SOWIE VERFAHREN ZUR HERSTELLUNG EINER DERARTIGEN MEMBRAN**
MEMBRANE FOR AN OXYGENATOR FOR GAS EXCHANGE IN THE BLOODSTREAM, OXYGENATOR HAVING SUCH A MEMBRANE, AND METHOD FOR PRODUCING SUCH A MEMBRANE
MEMBRANE DESTINÉE À UN OXYGÉNATEUR POUR ÉCHANGE GAZEUX DANS LA CIRCULATION SANGUINE, OXYGÉNATEUR POURVU D'UNE TELLE MEMBRANE ET PROCÉDÉ DE FABRICATION D'UNE TELLE MEMBRANE

(30) Priorität: 13.03.2015 DE 102015204638
(43) Veröffentlichungstag der Anmeldung: 17.01.2018
(73) Patentinhaber: Raumedic AG, 95213 Münchberg (DE)
(72) Erfinder: STÖCKER, Martin, 95233 Helmbrechts (DE)
(74) Vertreter: Rau, Schneck & Hübner Patentanwälte Rechtsanwälte PartGmbB
(86) Internationale Anmeldenummer: PCT/EP2016/055285
(87) Internationale Veröffentlichungsnummer: WO 2016/146524

(56) Entgegenhaltungen:
- EP-A1- 0 067 116
- EP-A1- 0 398 508
- EP-B1- 0 067 116
- WO-A1-2011/150216
- WO-A1-2012/098130
- DE-A1-102013 213 318
- GB-A- 2 086 762
- US-A- 3 727 612
- US-A- 3 892 533
- US-A- 4 666 668
- US-A1- 2009 078 644
- "PACO BASICS 3", 1 October 2017 (2017-10-01), PACO Paul GmbH & Co. KG
- "BOPP SD", 1 September 2017 (2017-09-01), G. Bopp + Co. KG
- Anonymous: "Fügen (Fertigungstechnik) - Wikipedia", , 6 March 2023 (2023-03-06), XP093029134, Retrieved from the Internet: URL:https://de.wikipedia.org/wiki/Fügen_(F ertigungstechnik) [retrieved on 2023-03-06]
- Anonymous: "Textiles Fügen - Wikipedia", , 6 March 2023 (2023-03-06), XP093029135, Retrieved from the Internet: URL:https://de.wikipedia.org/wiki/Textiles _Fügen [retrieved on 2023-03-06]

## Beschreibung

Die vorliegende Patentanmeldung nimmt die Priorität der deutschen Patentanmeldung DE 10 2015 204 638.9 in Anspruch.

Die Erfindung betrifft einen Oxygenator für Gasaustausch im Blutkreiskauf mit einer Membran.

Aus der EP 0 067 116 A1 ist eine Silikonkautschuk-Membran mit einem metallischen Stützgewebe bekannt.

Aus der DE 10 2013 213 318 A1 und aus der US 2009/0078664 A1 ist jeweils eine asymmetrisch poröse Membran aus aldehydvernetztem thermoplastischem Siliconelastomer bekannt. Die Membran weist Poren auf, die Hohlräume für den Austausch von Stoffen durch die Membran bilden. Um die Poren herstellen zu können, müssen zuvor im Membranmaterial vorhandene Bestandteile herausgelöst werden. Die Herstellung der Membran ist aufwendig. Das Membranmaterial ist für die Verwendung in einem Oxygenator aus Sicherheitsgründen nicht geeignet.

Aus der EP 2 109 492 B1 ist ein Oxygenator mit einer Membran bekannt, die aus einem Bündel von Silikonkautschuk-Schläuchen hergestellt ist. Die Herstellung der Membran erfordert eine Vielzahl von Fertigungsschritten. Fertigungsbedingt ist eine Mindestgröße des Oxygenators festgelegt. Für Anwendungen in kleinen Dimensionen, insbesondere für eine Anwendung bei Transplantaten, ist dieser Oxygenator nicht geeignet.

Weitere Oxygenatoren sind bekannt aus US 3,727,612, US 3,892,533 und GB 2 086 762 A.

EP 0 398 508 A1 offenbart eine Trennmembran für Pervaporationsverfahren.

WO 2012/098 130 A1 offenbart eine dreidimensionale Stützstruktur mit einem integrierten Permeationskanal.

WO 2011/150 216 A1 offenbart eine künstliche Lunge.

US 4,666,668 offenbart eine gasdurchlässige Membran für einen Oxygenator.

Es ist die Aufgabe der vorliegenden Erfindung, eine Membran für einen Oxygenator zu verbessern.

Diese Aufgabe ist erfindungsgemäß gelöst durch einen eine Membran aufweisenden Oxygenator mit den im Anspruch 1 angegebenen Merkmalen.

Erfindungsgemäß wurde erkannt, dass eine Membran eine Silikonlage und eine die Silikonlage verstärkende Verstärkungsstruktur aufweist, wobei die Silikonlage homogen ausgeführt ist. Homogen bedeutet, dass große Poren und/oder Lunker in der Silikonlage vermieden sind. Der Erfindung liegt die Erkenntnis zugrunde, dass Poren und/oder Lunker für den Gasaustausch durch die Membran nicht erforderlich sind. Der Gasaustausch kann durch Permeation durch die geschlossene Membran hindurch erfolgen. Die Silikonlage, insbesondere die Membran ist lunkerfrei. Dadurch ist die Gefahr von Plasmaleckagen während der Verwendung der Membran an einem Oxgenator ausgeschlossen. Es ist außerdem ausgeschlossen, dass in Folge unterschiedlich großer Poren, die beispielsweise bei einem Herstellungsverfahren gemäß der DE 10 2013 213 318 A1 auftreten können, Blut durch die poröse Membran durchdringen kann. Ein Bluttransfer durch die erfindungsgemäße Membran ist zuverlässig ausgeschlossen. Die Silikonlage ist insbesondere einteilig ausgeführt. Die Verstärkungsstruktur verleiht der Silikonlage, die insbesondere Silikonkautschuk aufweist (SIK), eine ausreichende Biegefestigkeit. Die Membran ist insgesamt stabil und vorteilhaft handhabbar. Die Verstärkungsstruktur verleiht der Membran eine ausreichende Eigensteifigkeit. Das bedeutet, dass die Membran nicht infolge ihres Eigengewichts ausknickt. Die Membran ist eine Gasaustauschmembran, die einen ersten Innenraum und einen zweiten Innenraum eines Oxygenators voneinander trennt. Die Gasaustauschmembran ist halb durchlässig, also semipermeabel. Die Gasaustauschmembran ist flüssigkeitsdicht, insbesondere blutdicht. Die Gasaustauschmembran ist gasdurchlässig, insbesondere ist eine Durchlässigkeit für Sauerstoff und Kohlendioxid gewährleistet. Die Verstärkungsstruktur ist insbesondere flächenhaft ausgeführt. Das bedeutet, dass eine Längendimension und eine Breitendimension der Verstärkungsstruktur mindestens eine Größenordnung größer sind als eine Dickendimension. Insbesondere betragen Länge und Breite der Verstärkungsstruktur mindestens das 10-fache der Dicke, insbesondere mindestens das 100-fache der Dicke und insbesondere höchstens das 1.000.000-fache der Dicke.

Bei der Membran weist die Verstärkungsstruktur einen Durchströmflächenanteil auf, der 98 % der Gesamtfläche der Verstärkungsstruktur beträgt, und gewährleistet, insbesondere auch bei kleiner Maschenweite der Verstärkungsstruktur, einen kleinen Flächenverbrauch. Insbesondere steht nahezu die Gesamtfläche der Verstärkungsstruktur als Durchströmflächenanteil zur Verfügung. Die Eignung als Gasaustauschmembran ist durch die Verwendung der Verstärkungsstruktur nicht nachteilig beeinträchtigt.

Ein Oxygenator mit der Membran weist im Wesentlichen die Vorteile der Membran selbst auf, worauf hiermit verwiesen wird. Der Oxygenator weist einen ersten Innenraum und einen davon durch die Membran getrennten zweiten Innenraum auf. Der erste Innenraum dient zum Durchströmen von Blut. Der zweite Innenraum dient zum Durchströmen von Gas, insbesondere Sauerstoff oder Kohlendioxid. Der erste Innenraum und der zweite Innenraum sind in einem Gehäuse angeordnet. Der Oxygenator ist für den Gasaustausch im Blutkreislauf, insbesondere im menschlichen Blutkreislauf, geeignet. Insbesondere ist eine Langzeitanwendung, die beispielsweise mindestens 30 Tage andauern kann, möglich, wie beispielsweise mobile Blutkreislaufunterstützungssysteme (ECLS und ECCOR) für Patienten mit akutem Herz- oder mit akutem Herzlungenversagen. Dadurch, dass insbesondere auf die Verwendung von Schlauchmaterial verzichtet werden kann, kann die Membran und somit der Oxygenator kleinbauend ausgeführt werden. Der Oxygenator kann als Mikro- Oxygenator ausgeführt sein. Aufgrund der reduzierten Baugröße des Oxygenators ist eine geringere Menge an Blut erforderlich, um den Oxygenator zu betreiben. Dies ist vorteilhaft. Zudem ist eine Schädigung des Blutes, das sich bei großbauenden Oxygenatoren infolge eines hohen Druckabfalls in dem Oxygenator und einer Verwirbelung des Blutes ergeben kann, ausgeschlossen. Eine Schädigung des Blutes kann beispielsweise durch eine Schädigung der Blutkörper selbst gegeben sein, die bis zur Hämolyse führen kann.

Eine Membran, bei der die Verstärkungsstruktur netzartig ausgeführt ist, vereinfacht die Bereitstellung der Verstärkungsstruktur. Die Verstärkungsstruktur selbst kann unkompliziert vorgefertigt sein. Die Verstärkungsstruktur weist Längselemente und Querelemente auf, die miteinander verbunden sind. Die Längselemente und die Querelemente sind insbesondere jeweils gleich beabstandet zueinander angeordnet. Die Verstärkungsstruktur weist ein regelmäßiges Raster, insbesondere ein Rechteckraster, und insbesondere ein Quadratraster auf. Die Verstärkungsstruktur ist eigensteif ausgeführt. Es ist grundsätzlich auch denkbar, eine Verstärkungsstruktur derart bereitzustellen, dass Verstärkungselemente lose, insbesondere als lose, ungeordnete Schüttung, insbesondere als Verstärkungsfasern, vorliegen, wobei die Verbindung der losen Verstärkungselemente mit der Silikonlage eine Fixierung der Verstärkungselemente einerseits und eine Verstärkung der Silikonlage andererseits bewirkt.

Eie Membran, bei der die Verstärkungsstruktur in die Silikonlage eingebettet ist, weist eine hohe Festigkeit auf. Die Verstärkungsstruktur ist insbesondere einseitig in die Silikonlage eingebettet. Das bedeutet, dass eine Unterseite der Verstärkungsstruktur vollständig von der Silikonlage umgeben ist. Eine der Unterseite abgewandte Oberseite der Verstärkungsstruktur kann freiliegen. Es ist auch denkbar, dass die Verstärkungsstruktur vollständig in der Silikonlage eingebettet ist.

Eine Membran, bei der die Verstärkungsstruktur Polyethersulfon (PES) aufweist, kann unmittelbar für medizinische Zwecke eingesetzt werden. Polyethersulfon ist medizinisch zugelassen. Grundsätzlich sind auch andere Materialien denkbar, die insbesondere eine medizinische Zulassung aufweisen, wie beispielsweise Polyester-Materialien. Wesentlich ist, dass das Material der Verstärkungsstruktur eine ausreichende Stabilität und/oder Formbarkeit aufweist, damit die Membran in dem Oxygenator zur Trennung der Innenräume einsetzbar ist. In Abhängigkeit der Bauform des Oxygenators kann es erforderlich sein, die Membran dreidimensional zu formen. Vorteilhaft ist es, wenn das Material der Verstärkungsstruktur biokompatibel ist. Eine Biokompatibilität ist aber nicht zwingend vorausgesetzt. Insbesondere dann, wenn die Verstärkungsstruktur vollständig in der Silikonlage eingebettet ist, kann auch ein Material, das nicht biokompatibel ist, eingesetzt werden. Insbesondere kann auch ein Geflecht aus Metalldraht für die Verstärkungsstruktur verwendet werden.

Eine Membran mit erhöhter Biegesteifigkeit hat eine verbesserte Stabilität. Die erhöhte Biegesteifigkeit bewirkt eine verringerte Elastizität in der Fläche der Membran, insbesondere in einer Längsrichtung und/oder in einer Querrichtung, die beide senkrecht zur Dickenrichtung der Membran orientiert sind.

Eine Membran, bei der die Verstärkungsstruktur eine Dicke von höchstens 0,4 mm, insbesondere von höchstens 0,35 mm und insbesondere von höchstens 0,3 mm aufweist, vereinfacht eine kleinbauende Ausführung des Oxygenators.

Eine Membran, bei der eine Dicke der Silikonlage zwischen 0,03 mm bis 0,5 mm, insbesondere zwischen 0,05 mm bis 0,4 mm und insbesondere zwischen 0,1 mm und 0,3 mm beträgt, ermöglicht eine dünnlagige Ausführung. Insbesondere beträgt die Dicke der Silikonlage maximal die Dicke der Verstärkungsstruktur. Die Dicke der Silikonlage kann in Abhängigkeit des Aufbaus der Membran auch größer sein als die Dicke der Verstärkungsstruktur.

Eine Membran, deren Dicke zwischen 0,35 mm bis 0,6 mm, insbesondere zwischen 0,4 mm bis 0,5 mm und insbesondere von 0,55 mm beträgt, kann vorteilhaft für kleinbauende Oxygenatoren, insbesondere für einen Mikro-Oxygenator, eingesetzt werden.

Ein Oxygenator mit einem äußeren Hohlzylinder aus einer ersten Membran und mit einem, in dem äußeren Hohlzylinder angeordneten inneren Hohlzylinder aus einer zweiten Membran ermöglicht eine vorteilhafte Ausführung. Der Oxygenator ist platzsparend und kompakt ausgeführt. Die Hohlzylinder sind insbesondere konzentrisch zu einer Längsachse des Gehäuses des Oxygenators angeordnet. Der erste Innenraum ist insbesondere zwischen einer Innenseite des äußeren Hohlzylinders und einer Außenseite des inneren Hohlzylinders begrenzt. Der erste Innenraum dient zum Durchströmen von Blut. Der erste Innenraum erstreckt sich also in radialer Richtung bezüglich der Längsachse. Der erste Innenraum ist ringförmig ausgeführt. Der zweite Innenraum ist von dem inneren Hohlzylinder begrenzt. Der zweite Innenraum dient zum Durchströmen von Gas. Eine Strömungsfläche senkrecht zu der Längsachse des inneren Hohlzylinders weist eine Kreisfläche auf. Insbesondere kann ein dritter Innenraum vorgesehen sein, der von einer Außenseite des äußeren Hohlzylinders und einer Innenseite des Gehäuses begrenzt ist. Der dritte Innenraum dient insbesondere für eine Durchströmung mit Gas. Dadurch ist ein Gasaustausch des Blutes zusätzlich verbessert.

Ein Oxygenator, bei dem eine erste Membran und eine zweite Membran vorgesehen sind, die jeweils bezüglich einer Längsachse des Gehäuses spiralförmig angeordnet sind, ermöglichen eine unkomplizierte Bauform. Der erste Innenraum ist durch die erste Membran und die zweite Membran begrenzt. Der Oxygenator kann vorteilhaft ein hohlzylindrisches Gehäuse aufweisen.

Ein Oxygenator, bei dem die erste Membran und die zweite Membran an einem Kern gehalten sind, ermöglicht eine zuverlässige und gezielte Anordnung der Membranen innerhalb des Gehäuses. Der Kern dient insbesondere als Abschluss des ersten, spiralförmig ausgeführten Innenraums. An einem anderen, dem Kern gegenüberliegenden Ende, kann der spiralförmige erste Innenraum mit einer Innenseite des Gehäuses verbunden sein. Der Kern ist insbesondere konzentrisch zur Längsachse angeordnet.

Ein Oxygenator, bei dem die Membran mäanderförmig angeordnet ist, ermöglicht eine besonders unkomplizierte Ausführung. Insbesondere ist genau eine Membran erforderlich, um den ersten Innenraum von dem zweiten Innenraum zu trennen. Der Oxygenator kann ein Gehäuse aufweisen, das senkrecht zu einer Längsachse eine rechteckförmige oder quadratische Form aufweist. Der erste Innenraum ist durch zwei benachbarte, insbesondere parallel orientierte, Lagen der Mäanderstruktur der Membran begrenzt. Benachbart zu dem ersten Innenraum ist der zweite Innenraum angeordnet. Der Oxygenator kann vorteilhaft mehrere erste Innenräume und/oder zweite Innenräume aufweisen, die insbesondere abwechselnd benachbart zueinander angeordnet sind. Die Schichtdicke des ersten Innenraums und/oder des zweiten Innenraums kann durch Abstandshalter vorgegeben sein. Es ist insbesondere denkbar, für den ersten Innenraum und den zweiten Innenraum Abstandshalter verschiedener Dicken vorzusehen.

Die Herstellung einer Membran für einen Oxygenator ist vereinfacht durch ein Verfahren mit den Verfahrensschritten Bereitstellen einer Verstärkungsstruktur, Umgießen der

Verstärkungsstruktur mit einer Silikondispersion und Vernetzen der Silikondispersion zu einer homogenen Silikonlage, in die die Verstärkungsstruktur eingebettet ist.

Es wurde erkannt, dass eine Membran für einen Oxygenator dadurch hergestellt werden kann, dass eine Verstärkungsstruktur von einer Silikondispersion umgossen wird, wobei anschließend die Silikondispersion zu einer homogenen Silikonlage vernetzt, in die Verstärkungsstruktur eingebettet ist. Die Verstärkungsstruktur ist zuverlässig in der Silikonlage gehalten. Die Silikonlage ist durch die Verstärkungsstruktur stabilisiert und dadurch handhabbar. Das Vernetzen der Silikondispersion erfolgt insbesondere in Abhängigkeit von Temperatur und/oder Zeit. Zusätzlich kann ein Trocknungsschritt vorgesehen sein. Das Trocknen kann während und/oder nach dem Vernetzen erfolgen. Ein nachträgliches, also nach dem Vernetzen durchzuführendes, herauslösen von Stoffen aus der Silikonlage, insbesondere aus dem Silikonkautschuk ist entbehrlich. Es ist nicht erforderlich, Lunker und/oder Poren in der Silikonlage zu bilden, um einen Materialstrom durch die Membran hindurch zu ermöglichen. Die erfindungsgemäße Membran ermöglicht einen Materialstrom, insbesondere einen Gasaustausch, durch Permeation durch die geschlossene Wand.

Ausführungsbeispiele der Erfindung werden nachfolgend anhand der Zeichnung näher erläutert. In dieser zeigen:
- Fig. 1: eine Seitenansicht einer erfindungsgemäßen Membran,
- Fig. 2: eine Schnittdarstellung gemäß Schnittlinie II-II in Fig. 1,
- Fig. 3: eine Ausführungsform eines Oxygenators mit einer erfindungsgemäßen Membran,
- Fig. 4: eine Querschnittsdarstellung gemäß der Schnittlinie IV-IV in Fig. 3,
- Fig. 5: eine Fig. 4 entsprechende Darstellung einer weiteren Ausführungsform eines Oxygenators mit spiralförmigen Membranen, und
- Fig. 6: eine Fig. 4 entsprechende Ansicht einer weiteren Ausführungsform eines Oxygenators mit mäanderförmig angeordneter Membran.

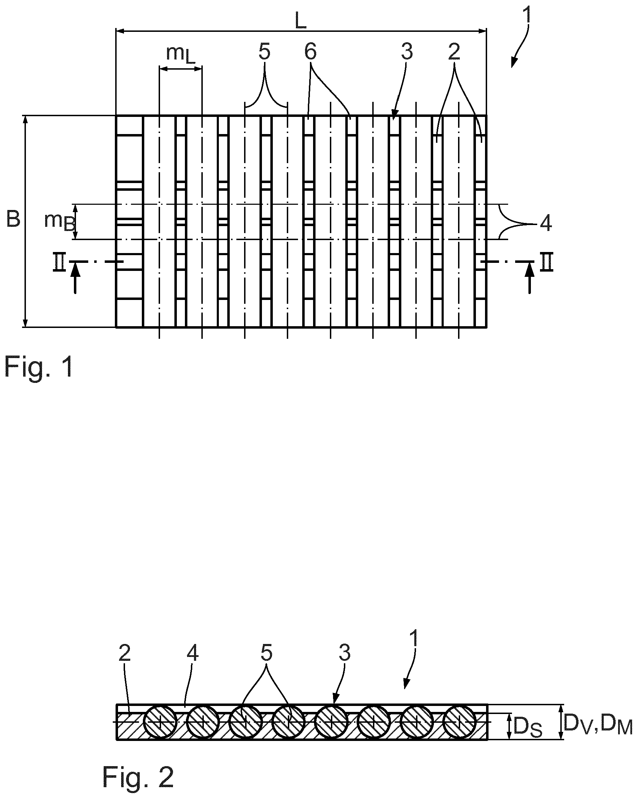

Eine in Fig. 1 und Fig. 2 dargestellte Membran 1 umfasst eine homogene, lunkerfreie Silikonlage 2 und eine die Silikonlage 2 verstärkende Verstärkungsstruktur 3.

Die Silikonlage 2 besteht insbesondere aus Silikonkautschuk. Die Silikonlage 2 verleiht der Membran 1 eine für den Einsatz in einem Oxygenator notwendige Semi-Permeablilität. Die Membran ist flüssigkeitsdicht, insbesondere blutdicht. Die Membran 1 ist gasdurchlässig, insbesondere durchlässig für Sauerstoff (O₂) und/oder Kohlendioxid (CO₂). Der verwendete Silikonwerkstoff weist eine gute Blutkompatibilität auf.

Die Silikonlage 2 ist flächenhaft ausgeführt. Flächenhaft bedeutet, dass Länge und Breite der Silikonlage 2 deutlich größer sind als eine Dicke Ds der Silikonlage 2. Die Dicke Ds beträgt gemäß dem gezeigten Ausführungsbeispiel 0,2 mm. Vorteilhaft ist es, wenn die Dicke Ds zwischen 0,1 mm und 0,3 mm beträgt. Insbesondere kann die Dicke Ds maximal im Bereich einer Dicke Dv der Verstärkungsstruktur 3 gewählt werden.

Die Verstärkungsstruktur 3 ist gemäß dem gezeigten Ausführungsbeispiel in die Silikonlage 2 einseitig eingebettet. Das bedeutet, dass die Verstärkungsstruktur 3 an einer Seite, insbesondere der Unterseite gemäß Fig. 2, in die Silikonlage 2 eingebettet ist. Die Unterseite der Verstärkungsstruktur 3 ist von der Silikonlage 2 abgedeckt. An einer der Unterseite gegenüberliegenden Oberseite liegt die Verstärkungsstruktur 3 zumindest abschnittsweise frei.

Es ist auch möglich, dass die Verstärkungsstruktur vollständig in der Silikonlage 2 eingebettet ist. Bei einer vollständigen Einbettung ist die Verstärkungsstruktur 3 vollständig von der Silikonlage 2 umgeben. Die Verstärkungsstruktur 3 ist dann vollständig innerhalb der Silikonlage 2, insbesondere vor äußeren Umgebungseinflüssen, geschützt angeordnet. Ein unmittelbarer Kontakt der Verstärkungsstruktur 3 mit dem Gas und/oder dem Blut in dem Oxygenator ist ausgeschlossen.

In diesem Fall, also bei vollständiger Einbettung der Verstärkungsstruktur 3, ist es unerheblich, ob die Verstärkungsstruktur 3 aus einem biokompatiblen Material hergestellt ist. Gemäß dem gezeigten Ausführungsbeispiel ist die Verstärkungsstruktur 3 aus biokompatiblem Polyethersulfon (PES) hergestellt. Die Verwendung eines biokompatiblen Materials für die Verstärkungsstruktur 3 ist vorteilhaft, wenn eine Beschädigung der Silikonlage 2 auftritt, sodass die Verstärkungsstruktur 3 unmittelbar mit dem Blut in Kontakt kommt.

Die Verstärkungsstruktur 3 ist netzartig ausgeführt. Die Verstärkungsstruktur 3 ist flächenhaft ausgeführt. Die Verstärkungsstruktur weist Längselemente 4 und Querelemente 5 auf. Die Längselemente 4 und die Querelemente 5 sind miteinander verbunden. Die Längselemente 4 und die Querelemente 5 sind jeweils als Verstärkungsfäden oder Verstärkungsfasern ausgeführt, die miteinander gefügt sind. Die Verstärkungsstruktur ist selbst stabil, weist also eine Eigensteifigkeit auf.

Gemäß dem gezeigten Ausführungsbeispiel beträgt die Dicke Dv der Verstärkungsstruktur etwa 0,2 mm. Es ist vorteilhaft, wenn die Dv höchstens 0,4 mm, insbesondere 0,25 mm und insbesondere 0,3 mm aufweist.

Die Längselemente 4 und die Querelemente 5 bilden ein quadratisches Raster mit einer Maschenweite m_{L} in Längsrichtung und einer Maschenweite m_{B} in Breitenrichtung. Gemäß dem gezeigten Ausführungsbeispiel gilt m_{L} = m_{B}. Die Maschenweiten m_{L} und m_{B} können auch unterschiedlich sein. Die Maschenweiten m_{L} und m_{B} betragen typischerweise 0,5 mm bis 5 mm. Es ist auch möglich, dass die Längselemente 4 und die Querelemente 5 nicht ortogonal zueinander orientiert sind.

Zwischen den Längselementen 4 und den Querelementen 5 der Verstärkungsstruktur 3 sind Zwischenräume 6 angeordnet. Die Dicke Dv der Verstärkungsstruktur, also die Dicken der Längselemente 4 und der Querelemente 5, ist derart gewählt, dass die Maschenweiten m_{L} und m_{B} größer sind als die Dicken der Längselemente 4 und der Querelemente 5. Insbesondere beträgt die Maschenweite m_{L} in Längsrichtung mindestens das Fünffache der Dicke der Querelemente 5 und insbesondere mindestens das Achtfache, insbesondere das Zehnfache. Die Maschenweite m_{B} in Breitenrichtung beträgt mindestens das Fünffache der Dicke der Längselemente 4, insbesondere mindestens das Achtfache und insbesondere mindestens das Zehnfache. Die Summe der Zwischenräume 6 bildet einen Durchströmflächenanteil der Verstärkungsstruktur 3. Der Durchströmflächenanteil beträgt 98 % der Gesamtfläche der Verstärkungsstruktur 3.

Die Gesamtfläche der Verstärkungsstruktur 3 ist insbesondere identisch mit der Gesamtfläche der Membran 1. Die Gesamtfläche der Membran 1 in Durchströmrichtung ist festgelegt durch die Länge L der Membran 1 und die Breite B der Membran 1. Die Durchströmrichtung ist die Dickenrichtung der Membran 1. In dem gezeigten Ausführungsbeispiel, bei dem die Membran 1 rechteckig ausgeführt ist, ergibt sich die Gesamtfläche als das Produkt aus Länge L und Breite B. Die Darstellung der Membran 1 in Fig. 1 und 2 ist nicht maßstäblich. Insbesondere ist aus darstellerischen Gründen die Verstärkungsstruktur 3, insbesondere die Längselemente 4 und die Querelemente 5, vergrößert, also mit vergrößerter Dicke, dargestellt. In der Realität kann die Dicke Dv der Verstärkungsstruktur auch reduziert sein.

Die Membran 1 weist eine Dicke D_{M} auf, die zwischen 0,35 mm und 0,6 mm beträgt. Gemäß dem gezeigten Ausführungsbeispiel beträgt die Dicke D_{M} etwa 0,45 mm.

Nachfolgend wird ein Verfahren zur Herstellung der Membran 1 näher erläutert. Zunächst wird die Verstärkungsstruktur 3 bereitgestellt. Die Verstärkungsstruktur 3 wird insbesondere auf einer nicht haftenden Unterlage aufgelegt. Eine nicht haftende Unterlage ist beispielsweise mit einer Oberfläche aus Polytetraflourethylen (PTFE) ausgeführt. Die Unterlage kann auch insgesamt aus PTFE hergestellt sein. Die Verstärkungsstruktur 3 wird mit einer Silikondispersion umgossen. Die Silikondispersion weist eine Viskosität von 80 mPas auf. Ziel ist es, eine möglicht dünne Silikonlage auszubilden. Je dünnflüssiger die Silikondispersion ist, also je geringer die Viskosität der Silikondispersion ist, desto dünner kann eine sich ausbildende Silikonlage ausgeführt sein. Insbesondere beträgt die Viskosität der Silikondispersion höchstens 200 mPas, insbesondere höchstens 100 mPas.

Nach dem Umgießen vernetzt die Silikondispersion zu der Silikonlage 2. Bei einer Temperatur von 50 °C erfolgt das Vernetzen der Silikondispersion innerhalb von etwa drei bis vier Stunden. Es ist möglich, eine höhere Vernetzungstemperatur vorzusehen. Die Vernetzungsdauer ist dann reduziert.

Zusätzlich zu dem Vernetzen kann ein Trocknungsschritt vorgesehen sein. Das Trocknen kann während und/oder nach dem Vernetzen erfolgen.

Das Verfahren zur Herstellung der Membran 1 ist insbesondere umweltfreundlich. Eine Mikroporösität, die beispielsweise bei anderen Membranwerkstoffen separat erzeugt werden muss, ist inhärent vorhanden. Ein zusätzlicher Herstellungsschritt, um die Mikroporösität zu erzeugen, ist entbehrlich.

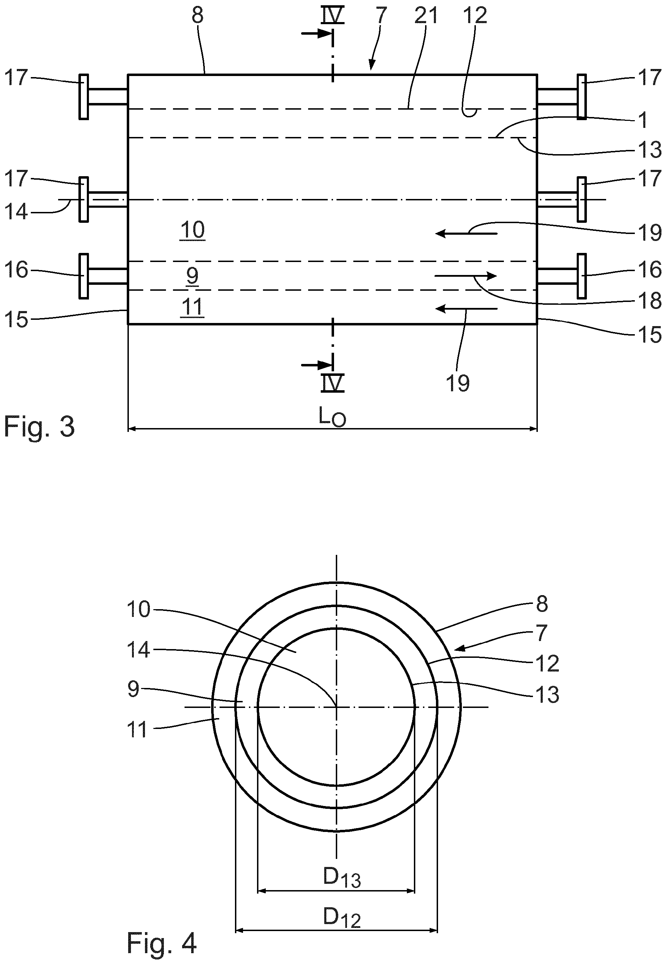

Eine in den Fig. 3 und 4 gezeigte Ausführungsform eines Oxygenators 7 dient für einen Gasaustausch im menschlichen Blutkreislauf. Der Oxygenator 7 umfasst ein Gehäuse 8, einen im Gehäuse 8 angeordneten ersten Innenraum 9 für Blut, einen in dem Gehäuse 8 angeordneten zweiten Innenraum 10 für Gas und einen in dem Gehäuse 8 angeordneten dritten Innenraum 11 für Gas. Der erste Innenraum 9 ist von dem zweiten Innenraum 10 durch eine erste Membran getrennt. Dazu ist die erste Membran ausgehend von der flächenhaften Struktur in Fig. 1 und 2 zu einem inneren Hohlzylinder 13 gedreht und insbesondere verklebt. Ferner ist eine zweite Membran vorgesehen, die in analoger Weise zu einem äußeren Hohlzylinder 12 gedreht und verklebt ist.

Das Gehäuse 8 ist hohlzylindrisch ausgeführt und weist eine Längsachse 14 auf. Der innere Hohlzylinder 13 ist innerhalb des äußeren Hohlzylinders 12 angeordnet. Der innere Hohlzylinder 13 und der äußere Hohlzylinder 12 sind konzentrisch zur Längsachse 14 in dem Gehäuse 8 angeordnet. Der erste Innenraum 9 ist in radialer Richtung bezüglich der Längsachse 14 zwischen dem inneren Hohlzylinder 13 und dem äußeren Hohlzylinder 12 begrenzt. Der erste Innenraum 9 ist von einer Innenseite des äußeren Hohlzylinders 12 und einer Außenseite des inneren Hohlzylinders 13 in radialer Richtung begrenzt.

In axialer Richtung der Längsachse 14 ist der erste Innenraum 9 mittels stirnseitiger Deckel 15 begrenzt. Der erste Innenraum 9 ist über BlutAnschlüsse 16 mit Verbindungsleitungen verbindbar. Die Blutanschlüsse 16 weisen insbesondere eine Luer-Lock-Verbindung auf. Jeweils ein Blut-anschluss 16 ist an einem Deckel 15 angeordnet. Blut kann von dem einen Blutanschluss 16 durch den ersten Innenraum, also im Wesentlichen entlang der Längsachse 14, zu dem jeweils anderen Blutanschluss 16 strömen.

Der zweite Innenraum 10 ist in radialer Richtung durch den inneren Hohlzylinder 13 begrenzt. Der zweite Innenraum 10 weist eine senkrecht zur Längsachse 14 orientierte kreisförmige Fläche auf. Der erste Innenraum 9 und der dritte Innenraum 11 weisen jeweils eine ringflächenförmige Querschnittsfläche senkrecht zur Längsachse 14 auf. In axialer Richtung ist der zweite Innenraum 10 durch die Deckel 15 begrenzt. Der zweite Innenraum 10 ist für Gas-Anschlüsse 17, die jeweils an dem Deckel 15 angeordnet sind, mit Verbindungsleitungen verbindbar. Die Gas-Anschlüsse 17 weisen eine Luer-Verbindung auf, an der insbesondere eine Luer-Lock-Sperre vorgesehen ist. Wesentlich ist, dass Anschlusselemente der Blut-Anschlüsse 16 und der Gas-Anschlüsse 17 verschieden ausgeführt sind, sodass zuverlässig gewährleistet ist, dass ein versehentliches Anschließen einer Blut-Verbindungsleitung an den Gas-Anschluss 17 beziehungsweise einer Gas-Verbindungsleitung an den Blut-Anschluss 16 ausgeschlossen ist. Eine verschiedenartige Ausführung kann auch dadurch gewährleistet sein, dass der Blut-Anschluss 16 eine Gewinde-Nase zum Verbinden mit einer korrespondierenden Mutter aufweist. In diesem Fall weisen die Gas-Anschlüsse 17 keinen Gewindeanschluss auf. Eine Fehlanwendung des Oxygenators 7 ist verhindert.

Der dritte Innenraum 11 ist in radialer Richtung durch eine Außenseite des äußeren Hohlzylinders 12 und eine Innenseite des Gehäuses 8 begrenzt. In axialer Richtung ist der dritte Innenraum 11 durch die Deckel 15 begrenzt. Der dritte Innenraum 11 ist mittels Gas-Anschlüssen 17, die insbesondere identisch sind zu den Gas-Anschlüssen 17 des zweiten Innenraums 10, mit Verbindungsleitungen verbindbar.

Das Gehäuse 8 kann auch zwei Gehäusehälften aufweisen, die unlösbar, beispielsweise durch Kleben oder Umgießen, miteinander verbunden sind. Typischerweise ist eine Trennebene von zwei Gehäusehälften entsprechend der Schnittlinie IV-IV in Fig. 3 orientiert. Eine Trennebene ist also senkrecht zur Längsachse 14 orientiert. Der Oxygenator 7 garantiert, dass ein direkter Kontakt zwischen Blut in dem ersten Innenraum 9 und Sauerstoff beziehungsweise Kohlendioxid in dem zweiten und dritten Innenraum 10, 11 erfolgt. Der Oxygenator 7 weist eine reduzierte Baugröße auf. Die Länge Lo des Oxygenators entlang der Längsachse 14 beträgt gemäß dem gezeigten Ausführungsbeispiel 36 mm. Die Länge Lo des Oxygenators 7 ist insbesondere identisch zu der Breite B der Membran 1. Ein Durchmesser D₁₃ des inneren Hohlzylinders 13 beträgt gemäß dem gezeigten Ausführungsbeispiel 21 mm. Der Durchmesser D₁₂ des äußeren Hohlzylinders 12 beträgt gemäß dem gezeigten Ausführungsbeispiel 27 mm.

Insbesondere wird der Oxygenator 7 dadurch betrieben, dass Blut durch den ersten Innenraum 9 entlang einer Blutströmungsrichtung 18 strömt. Die Blutströmungsrichtung 18 ist in Fig. 3 von links nach rechts orientiert. Sauerstoff und Kohlendioxid strömen durch den zweiten Innenraum 10 beziehungsweise den dritten Innenraum 11 entlang einer Gasströmungsrichtung 19. Die Gasströmungsrichtung 19 ist der Blutströmungsrichtung 18 entgegengerichtet. Die Gasströmungsrichtung 19 und die Blutströmungsrichtung 18 sind antiparallel. In Fig. 3 ist die Gasströmungsrichtung von rechts nach links orientiert.

Nachfolgend wird das Verfahren zur Herstellung des Oxygenators 7 näher erläutert. Zunächst wird die erste Membran zu dem äußeren Hohlzylinder 12 gedreht und verklebt. Dabei bildet die Länge L der Membran 1 im Wesentlichen die Umfangslinie des äußeren Hohlzylinders 12. Anschließend wird der innere Hohlzylinder 13 aus der zweiten Membran gedreht und verklebt. Der innere Hohlzylinder 13 wird konzentrisch zu dem äußeren Hohlzylinder 12 angeordnet. Anschließend werden die beiden Hohlzylinder 12, 13 mit dem Gehäuse 8 unlösbar gefügt. Dies kann beispielsweise dadurch erfolgen, dass das hohlzylindrische Gehäuse 8 mit einem der stirnseitigen Deckel 15 verbunden, insbesondere geklebt, wird. Anschließend werden die Hohlzylinder 12, 13 in das Gehäuse 8 eingeführt und stirnseitig mit dem Deckel 15 verbunden. Dieses Verbinden erfolgt entweder durch Eingießen oder Kleben. Insbesondere wird für das Eingießen der Hohlzylinder 12, 13 das gleiche Material verwendet wie für das Verkleben der Hohlzylinder 12, 13. Anschließend kann der zweite stirnseitige Deckel 15 an das Gehäuse 8 angesetzt und mit dem Gehäuse 8 und Hohlzylindern 12, 13 verbunden werden.

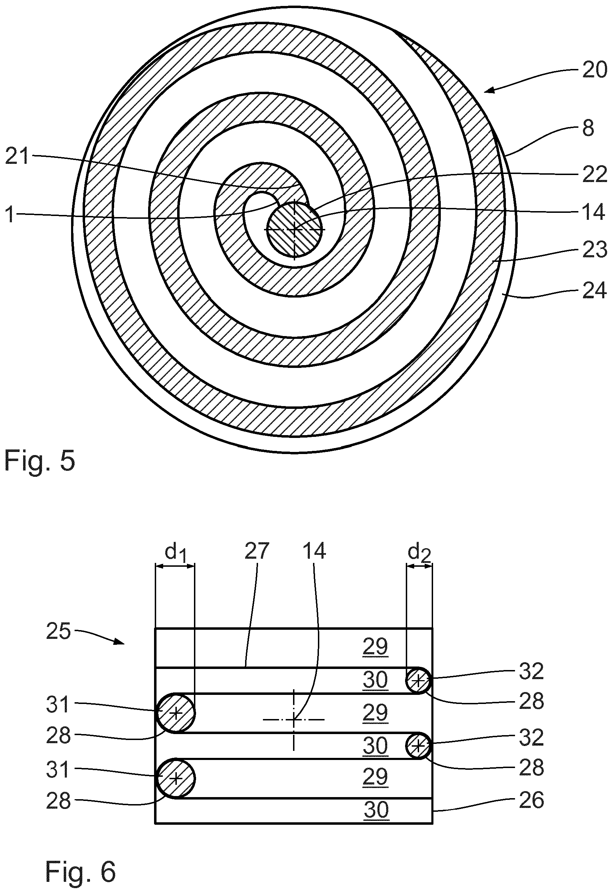

Fig. 5 zeigt eine weitere Ausführung eines Oxygenators 20. Komponenten, die denjenigen entsprechen, die vorstehend unter Bezugnahme auf die Fig. 1 bis 4 bereits erläutert wurden, tragen die gleichen Bezugsziffern und werden nicht nochmals im Einzelnen diskutiert.

Der wesentliche Unterschied des Oxygenators 20 gegenüber der vorherigen Ausführungsform besteht darin, dass die erste Membran 1 und die zweite Membran 21 spiralförmig bezüglich der Längsachse 14 angeordnet sind. Die erste Membran 1 und die zweite Membran 21 sind an einem jeweils ersten Ende an einem Kern 22 befestigt. Der Kern 22 ist konzentrisch zur Längsachse 14 in dem Gehäuse 8 angeordnet. Die jeweils gegenüberliegenden Enden der Membranen 1 und 21 sind an einer Innenseite des Gehäuses 8 befestigt.

Infolge der spiralförmigen Anordnung der Membranen 1, 21 ist der erste Innenraum 23 spiralförmig ausgeführt. Der zweite Innenraum 24 ist ebenfalls spiralförmig ausgeführt. Ein dritter Innenraum ist bei dem Oxygenator 20 nicht vorgesehen. Allein aus Darstellungsgründen ist der erste Innenraum 23 gemäß dem gezeigten Ausführungsbeispiel schraffiert dargestellt. Es ist klar, dass der erste Innenraum 23 hohl ausgeführt ist.

An jeweils stirnseitigen Deckeln weist der Oxygenator 20 jeweils einen Blut-Anschluss und einen Gas-Anschluss auf.

Fig. 6 zeigt eine weitere Ausführungsform eines Oxygenators 25. Komponenten, die denjenigen entsprechen, die vorstehend unter Bezugnahme auf die Fig. 1 bis 5 erläutert wurden, tragen die gleichen Bezugsziffern und werden nicht nochmals im Einzelnen diskutiert.

Der wesentliche Unterschied gegenüber den vorherigen Ausführungen besteht darin, dass genau eine Membran 1 vorgesehen ist. Die Membran ist mäanderförmig in dem Gehäuse 26 angeordnet. Die Mäanderstruktur ist in der Schnittebene gemäß Fig. 6 dargestellt. Das bedeutet, dass Lagen 27 der Mäanderstruktur jeweils parallel zu der Längsachse 14 des Oxygenators 25 orientiert sind. Die Lagen 27 sind insbesondere parallel zueinander orientiert. Zwei parallele, benachbarte Lagen 27 sind über einen U-förmigen Umlenkabschnitt 28 einstückig miteinander verbunden. Die Umlenkabschnitte 28 sind insbesondere jeweils an einer Außenseite mit einer Innenseite des Gehäuses 26 fest verbunden. Die Membran 1 trennt jeweils einen ersten Innenraum 29 von einem benachbarten zweiten Innenraum 30. Gemäß dem gezeigten Ausführungsbeispiel sind drei erste Innenräume 29 und drei zweite Innenräume 30 vorgesehen, wobei die Innenräume 29, 30 in abwechselndem Schichtaufbau angeordnet sind.

An den jeweiligen Umlenkabschnitten 28 sind erste Rollen 31 und zweite Rollen 32 vorgesehen. Die ersten Rollen 31 sind jeweils innerhalb des ersten Innenraums 29 angeordnet. Die ersten Rollen 31 gewährleisten, dass benachbarte Lagen 27 mit einem definierten Abstand zueinander angeordnet sind. Der definierte Abstand der benachbarten Lagen 27 wird durch den Durchmesser der ersten Rollen 31 vorgegeben. Die erste Rolle 31 weist einen ersten Durchmesser d₁ auf. Der erste Durchmesser d₁ beträgt höchstens 3 mm, insbesondere höchstens 2,5 mm.

Entsprechend weist die zweite Rolle 32 einen zweiten Durchmesser d₂ auf. Der zweite Durchmesser d₂ ist kleiner als der erste Durchmesser d₁. Insbesondere beträgt der zweite Durchmesser d₂ weniger als 2,5 mm, insbesondere weniger als 2 mm und insbesondere weniger als 1,5 mm.

Das Gehäuse 26 weist eine rechteckigförmige Kontur auf. Der Oxygenator 25 weist eine senkrecht zur Zeichenebene der Fig. 6 orientierte, also entlang der Längsachse 14 gerichtete, Länge von etwa 15 cm auf. Aufgrund der kompakten und mehrlagigen Bauweise des Oxygenators 25 mit der erfindungsgemäßen Membran 1 ermöglicht dieser einen verbesserten Gasaustausch.

## Patentansprüche

1. Oxygenator (7; 20; 25) für Gasaustausch im Blutkreislauf umfassend
a. ein Gehäuse (8; 26),
b. einen in dem Gehäuse (8; 26) angeordneten ersten Innenraum (9; 23; 29) für Blut,
c. einen in dem Gehäuse (8; 26) angeordneten zweiten Innenraum (10; 24; 30) für Gas,
d. eine den ersten Innenraum (9; 23; 29) und den zweiten Innenraum (10; 24; 30) trennende Membran (1, 21; 1, 21; 1) umfassend eine Silikonlage (2) und eine die Silikonlage (2) verstärkende Verstärkungsstruktur (3),
wobei die Silikonlage (2) homogen ausgeführt ist,
**dadurch gekennzeichnet, dass** die Verstärkungsstruktur (3) netzartig und flächenhaft ausgeführt ist und Längselemente (4) und Querelemente (5) aufweist, wobei die Längselemente (4) und die Querelemente (5) jeweils als Verstärkungsfäden oder Verstärkungsfasern ausgeführt sind, die miteinander gefügt sind, sodass die Verstärkungsstruktur (3) eine Eigensteifigkeit aufweist, wobei die Verstärkungsstruktur (3) eine Dicke (Dᵥ) aufweist, die den Dicken der Längselemente (4) und der Querelemente (5) entspricht und derart gewählt ist, dass eine Maschenweite (m_{L}) in Längsrichtung und eine Maschenweite (m_{B}) in Breitenrichtung größer sind als die Dicken der Längselemente (4) und der Querelemente (5), wobei die Maschenweite (m_{B}) in Breitenrichtung mindestens das fünffache der Dicke der Längselemente (4) beträgt, wobei zwischen den Längselementen (4) und den Querelementen (5) der Verstärkungsstruktur (3) Zwischenräume (6) angeordnet sind, wobei die Summe der Zwischenräume (6) einen Durchströmflächenanteil der Verstärkungsstruktur (3) bildet, wobei der Durchströmflächenanteil 98 % der Gesamtfläche der Verstärkungsstruktur (3) beträgt, wobei eine Durchströmrichtung die Dickenrichtung der Membran (1) ist.

2. Oxygenator gemäß einem der vorstehenden Ansprüche, **dadurch gekennzeichnet, dass** die Verstärkungsstruktur (3), insbesondere einseitig, in der Silikonlage (2) eingebettet ist.

3. Oxygenator gemäß einem der vorstehenden Ansprüche, **dadurch gekennzeichnet, dass** die Verstärkungsstruktur (3) Polyethersulfon (PES) aufweist.

4. Oxygenator gemäß einem der vorstehenden Ansprüche, **dadurch gekennzeichnet, dass** die Dicke (Dv) der Verstärkungsstruktur (3) von höchstens 0,4 mm, insbesondere von höchstens 0,35 mm und insbesondere von höchstens 0,3 mm beträgt.

5. Oxygenator gemäß einem der vorstehenden Ansprüche, **dadurch gekennzeichnet, dass** eine Dicke (Ds) der Silikonlage (2) zwischen 0,03 mm bis 0,5 mm, insbesondere zwischen 0,05 mm bis 0,4 mm und insbesondere zwischen 0,1 mm bis 0,3 mm beträgt.

6. Oxygenator gemäß einem der vorstehenden Ansprüche, **dadurch gekennzeichnet, dass** eine Dicke (D_{M}) der Membran (1) zwischen 0,35 mm bis 0,6 mm, insbesondere zwischen 0,4 mm bis 0,5 mm und insbesondere etwa 0,45 mm beträgt.

7. Oxygenator (7) gemäß einem der vorherigen Ansprüche , **gekennzeichnet durch** einen äußeren Hohlzylinder (12) aus einer ersten Membran (1) und einen, in dem äußeren Hohlzylinder (12) angeordneten inneren Hohlzylinder (13) aus einer zweiten Membran (21), wobei der erste Innenraum (9) von einer Innenseite des äußeren Hohlzylinders (12) und einer Außenseite des inneren Hohlzylinders (13) begrenzt ist, wobei der zweite Innenraum (10) von dem inneren Hohlzylinders (13) begrenzt ist und wobei insbesondere ein dritter Innenraum (11) vorgesehen ist, der von einer Innenseite des Gehäuses (8) und einer Außenseite des äußeren Hohlzylinders (12) begrenzt ist.

8. Oxygenator (20) gemäß einem der Ansprüche 1 bis 6, **gekennzeichnet durch** eine erste Membran (1) und eine zweite Membran (21), die jeweils bezüglich einer Längsachse (14) spiralförmig angeordnet sind, wobei der erste Innenraum (23) durch die erste Membran (1) und die zweite Membran (21) begrenzt ist.

9. Oxygenator (20) gemäß Anspruch 8, **dadurch gekennzeichnet, dass** die erste Membran (1) und die zweite Membran (21) an einem Kern (22) gehalten sind, der insbesondere konzentrisch zur Längsachse (14) angeordnet ist.

10. Oxygenator (25) gemäß einem der vorherigen Ansprüche, **dadurch gekennzeichnet, dass** die Membran (1) mäanderförmig angeordnet ist, wobei der erste Innenraum (29) durch zwei benachbarte Lagen (27) der Mäanderstruktur der Membran (1) begrenzt ist und wobei benachbart zu dem ersten Innenraum (29) der zweite Innenraum (30) angeordnet ist.

## Claims

1. Oxygenator (7; 20; 25) for gas exchange in the blood-circulation system comprising
a. a housing (8; 26),
b. arranged in the housing (8; 26), a first internal chamber (9; 23; 29) for blood,
c. arranged in the housing (8; 26), a second internal chamber (10; 24; 30) for gas,
d. a membrane (1, 21; 1, 21; 1) separating the first internal chamber (9; 23; 29) and the second internal chamber (10; 24; 30), comprising a silicone layer (2) and a reinforcing structure (3) which reinforces the silicone layer (2), wherein the silicone layer (2) is homogeneous,
**characterized in that** the reinforcing structure (3) takes the form of a network and an area and has longitudinal elements (4) and transverse elements (5), wherein the longitudinal elements (4) and the transverse elements (5) take the form of reinforcing filaments or reinforcing fibres which have been joined to one another such that the reinforcing structure has an intrinsic rigidity, wherein the reinforcing structure (3) has a thickness (Dv) which corresponds to the thicknesses of the longitudinal elements (4) and the transverse elements (5) and is selected such that a mesh width (m_{L}) in the longitudinal direction and a mesh width (m_{B}) in the transverse direction are greater than thicknesses of the longitudinal elements (4) and of the transverse elements (5), wherein the mesh width (ms) in the transverse direction is at least five times the thickness of the longitudinal elements (4), wherein intervening spaces (6) are arranged between the longitudinal elements (4) and the transverse elements (5) of the reinforcing structure (3), wherein the totality of the intervening spaces (6) forms an area available for flow through the reinforcing structure (3), wherein the area available for flow is in particular at least 98% of the total area of the reinforcing structure (3), wherein the direction of flow is the direction of thickness of the membrane (1).

2. Oxygenator according to any one of the preceding claims, **characterized in that** the reinforcing structure (3), has been embedded into the silicone layer (2), in particular on one side.

3. Oxygenator according to any one of the preceding claims, **characterized in that** the reinforcing structure (3) comprises polyether sulfone (PES).

4. Oxygenator according to any one of the preceding claims, **characterized in that** the thickness (Dv) of the reinforcing structure (3) is at most 0.4 mm, in particular at most 0.35 mm and in particular at most 0.3 mm.

5. Oxygenator according to any one of the preceding claims, **characterized in that** a thickness (D_{S}) of the silicone layer (2) is from 0.03 mm to 0.5 mm, in particular from 0.05 mm to 0.4 mm and in particular from 0.1 mm to 0.3 mm.

6. Oxygenator according to any one of the preceding claims, **characterized in that** a thickness (D_{M}) of the membrane (1) is from 0.35 mm to 0.6 mm, in particular from 0.4 mm to 0.5 mm and in particular about 0.45 mm.

7. Oxygenator (7) according to any one of the preceding claims, **characterized by** an exterior hollow cylinder (12) made of a first membrane (1) and, arranged in the exterior hollow cylinder (12), an interior hollow cylinder (13) made of a second membrane (21), where the first internal chamber (9) is delimited by an internal side of the exterior hollow cylinder (12) and by an external side of the interior hollow cylinder (13), where the second internal chamber (10) is delimited by the interior hollow cylinder (13), and where in particular there is a third internal chamber (11) provided which is delimited by an internal side of the housing (8) and by an external side of the exterior hollow cylinder (12).

8. Oxygenator (20) according to any one of claims 1 to 6, **characterized by** a first membrane (1) and a second membrane (21), respectively arranged spirally with respect to a longitudinal axis (14), where the first internal chamber (23) is delimited by the first membrane (1) and by the second membrane (21).

9. Oxygenator (20) according to claim 8, **characterized in that** the first membrane (1) and the second membrane (21) are retained on a core (22) which in particular is arranged concentrically with respect to the longitudinal axis (14).

10. Oxygenator (25) according to any one of the preceding claims, **characterized in that** the arrangement of the membrane (1) is in a meandering pattern, where the first internal chamber (29) is delimited by two adjacent layers (27) of the meandering structure of the membrane (1), and where the second internal chamber (30) is arranged adjacent to the first internal chamber (29).

## Revendications

1. Oxygénateur (7 ; 20 ; 25) pour l'échange de gaz dans la circulation sanguine, comprenant
a. un boîtier (8 ; 26),
b. un premier espace intérieur (9 ; 23 ; 29) pour le sang, disposé dans le boîtier (8 ; 26),
c. un deuxième espace intérieur (10 ; 24 ; 30) pour le gaz, disposé dans le boîtier (8 ; 26),
d. une membrane (1, 21 ; 1, 21 ; 1) séparant le premier espace intérieur (9 ; 23 ; 29) et le deuxième espace intérieur (10 ; 24 ; 30), comprenant une couche de silicone (2) et une structure de renforcement (3) renforçant la couche de silicone (2),
la couche de silicone (2) étant réalisée de manière homogène,
**caractérisé en ce que** la structure de renforcement (3) est réalisée sous forme de réseau et de surface et présente des éléments longitudinaux (4) et des éléments transversaux (5), les éléments longitudinaux (4) et les éléments transversaux (5) étant réalisés respectivement sous forme de fils de renforcement ou de fibres de renforcement, qui sont assemblés entre eux de sorte que la structure de renforcement (3) présente une rigidité propre, la structure de renforcement (3) présentant une épaisseur (Dv) qui correspond aux épaisseurs des éléments longitudinaux (4) et des éléments transversaux (5) et qui est choisie de telle sorte, qu'une largeur de maille (m_{L}) dans la direction longitudinale et une largeur de maille (ms) dans la direction de la largeur sont supérieures aux épaisseurs des éléments longitudinaux (4) et des éléments transversaux (5), la largeur de maille (m_{B}) dans la direction de la largeur étant égale à au moins cinq fois l'épaisseur des éléments longitudinaux (4), des espaces intermédiaires (6) étant disposés entre les éléments longitudinaux (4) et les éléments transversaux (5) de la structure de renforcement (3), la somme des espaces intermédiaires (6) formant un pourcentage de surface d'écoulement de la structure de renforcement (3), le pourcentage de surface d'écoulement étant de 98 % de la surface totale de la structure de renforcement (3), une direction d'écoulement étant la direction de l'épaisseur de la membrane (1).

2. Oxygénateur selon l'une quelconque des revendications précédentes, **caractérisé en ce que** la structure de renforcement (3) est noyée, en particulier d'un seul côté, dans la couche de silicone (2).

3. Oxygénateur selon l'une quelconque des revendications précédentes, **caractérisé en ce que** la structure de renforcement (3) comprend du polyéthersulfone (PES).

4. Oxygénateur selon l'une quelconque des revendications précédentes, **caractérisé en ce que** l'épaisseur (Dv) de la structure de renforcement (3) est d'au plus 0,4 mm, en particulier d'au plus 0,35 mm et en particulier d'au plus 0,3 mm.

5. Oxygénateur selon l'une quelconque des revendications précédentes, **caractérisé en ce qu'**une épaisseur (Ds) de la couche de silicone (2) est comprise entre 0,03 mm et 0,5 mm, en particulier entre 0,05 mm et 0,4 mm, et en particulier entre 0,1 mm et 0,3 mm.

6. Oxygénateur selon l'une quelconque des revendications précédentes, **caractérisé en ce qu'**une épaisseur (D_{M}) de la membrane (1) est comprise entre 0,35 mm et 0,6 mm, en particulier entre 0,4 mm et 0,5 mm, et en particulier d'environ 0,45 mm.

7. Oxygénateur (7) selon l'une quelconque des revendications précédentes, **caractérisé par** un cylindre creux extérieur (12) constitué d'une première membrane (1) et un cylindre creux intérieur (13) constitué d'une deuxième membrane (21), disposé dans le cylindre creux extérieur (12), le premier espace intérieur (9) étant délimité par une face intérieure du cylindre creux extérieur (12) et une face extérieure du cylindre creux intérieur (13), le deuxième espace intérieur (10) étant délimité par le cylindre creux intérieur (13) et en particulier un troisième espace intérieur (11) étant prévu, qui est délimité par une face intérieure du boîtier (8) et une face extérieure du cylindre creux extérieur (12).

8. Oxygénateur (20) selon l'une quelconque des revendications 1 à 6, **caractérisé par** une première membrane (1) et une deuxième membrane (21) disposées chacune en spirale par rapport à un axe longitudinal (14), le premier espace intérieur (23) étant délimité par la première membrane (1) et la deuxième membrane (21).

9. Oxygénateur (20) selon la revendication 8, **caractérisé en ce que** la première membrane (1) et la deuxième membrane (21) sont maintenues sur un noyau (22), qui est en particulier disposé de manière concentrique par rapport à l'axe longitudinal (14).

10. Oxygénateur (25) selon l'une quelconque des revendications précédentes, **caractérisé en ce que** la membrane (1) est disposée en méandres, le premier espace intérieur (29) étant délimité par deux couches adjacentes (27) de la structure en méandres de la membrane (1) et le deuxième espace intérieur (30) étant disposé au voisinage du premier espace intérieur (29).
